# EUROPEAN PATENT APPLICATION

(11) **EP 2 567 652 A1**
(43) Date of publication of application: **13.03.2013**
(21) Application number: 11814365.0
(22) Date of filing: 06.06.2011
(51) Int. Cl.: A61B 1/04

(54) **ENDOSCOPE SYSTEM**

(30) Priority: 02.08.2010 JP 2010173862
(71) Applicant: Olympus Medical Systems Corporation, Shibuya-ku Tokyo 151-0072 (JP)
(72) Inventor: KUTSUMA Yuji, Tokyo 151-0072 (JP)
(74) Representative: Gunzelmann, Rainer
(86) International application number: PCT/JP2011/062955
(87) International publication number: WO 2012/017735

(57) **Abstract**

An endoscope system comprises: an endoscope which outputs a video signal based on an endoscope image obtained by an image pickup section for picking up an optical image of a subject; and a video processor to which the endoscope is detachably connected and which performs video signal processing on the video signal from the endoscope. The video processor comprises a correction amount acquiring section, in a case where the endoscope has a first frequency correction section for performing a frequency correction of the video signal, to which first frequency correction information used in the first frequency correction section is inputted and which acquires second frequency correction information for performing a frequency correction in concert with frequency correction processing by the first frequency correction section based on the first frequency correction information, and a second frequency correction section which performs the frequency correction on the video signal from the endoscopes using the second frequency correction information.

## Description

### Technical Field

The present invention relates to an endoscope system for processing an endoscope image from an image pickup section.

### Background Art

In recent years, endoscopes (hereinafter referred to as scopes) have been widely used for diagnosis, treatment using a treatment instrument, and the like in a medical field. Electronic endoscope apparatuses which have an image pickup device such as a charge coupled device (CCD) provided at an endoscope distal end and display an observed image picked up using the CCD on a TV monitor by a video processor are in widespread use.

A video processor subjects a video signal from a scope to various types of video signal processing and outputs the processed signal to a monitor. For example, in the video processor, correction processing such as edge enhancement processing is performed.

In some endoscope systems, a correction circuit (hereinafter referred to as an in-scope correction circuit) for correction processing on a video signal read out from an image pickup device provided at an insertion section distal end is provided as an integrated circuit in a scope. The video signal from the image pickup device is subjected to correction processing in the in-scope correction circuit and is then outputted to a video processor.

A scope incorporating a CDS circuit which performs CDS (correlated double sampling) processing on an output from an image pickup device has also been developed (see Japanese Patent Application Laid-Open Publication No. 2008-80007). In the scope, a video signal having undergone CDS processing is largely affected by cable transmission characteristics in the scope to increase attenuation of a high-frequency range component of an image. For the reason, the scope is configured to subject an output from the CDS circuit to correction processing by an in-scope correction circuit and then output a result to a video processor.

However, as mentioned above, the correction processing such as edge enhancement processing is also performed in the video processor, and there has been a problem that the video signal is deteriorated by the correction processing in the video processor under the influence of the correction processing by the in-scope correction circuit.

An object of the present invention is to provide an endoscope system capable of improving image quality by performing correction processing in an in-processor correction circuit in concert with correction processing by an in-scope correction circuit.

### Disclosure of Invention

### Means for Solving the Problem

An endoscope system according to one aspect of the present invention comprises: an endoscope which outputs a video signal based on an endoscope image obtained by an image pickup section for picking up an optical image of a subject; and a video processor to which the endoscope is detachably connected and which performs video signal processing on the video signal from the endoscope, wherein the video processor comprises a correction amount acquiring section, in a case where the endoscope has a first frequency correction section for performing a frequency correction of the video signal, to which first frequency correction information used in the first frequency correction section is inputted and which acquires second frequency correction information for performing a frequency correction in concert with frequency correction processing by the first frequency correction section based on the first frequency correction information, and a second frequency correction section which performs the frequency correction on the video signal from the endoscopes using the second frequency correction information.

### Brief Description of the Drawings

Fig. 1 is a block diagram showing an endoscope system according to a first embodiment of the present invention.
Fig. 2 is a circuit diagram showing a specific configuration of a frequency correction section 16 of an in-scope substrate 15 in Fig. 1.
Fig. 3 is a waveform chart for explaining action of the frequency correction section 16.
Fig. 4 is an explanatory view for explaining operation of a white spot correction circuit 23.
Fig. 5 is a flow chart showing an operation flow of the white spot correction circuit 23.
Fig. 6 is a graph showing transient response characteristics, with time on an abscissa and a controlled variable on an ordinate.
Fig. 7 is a block diagram showing a second embodiment of the present invention.

### Best Mode for Carrying Out the Invention

Embodiments of the present invention will be described below in detail with reference to the drawings.

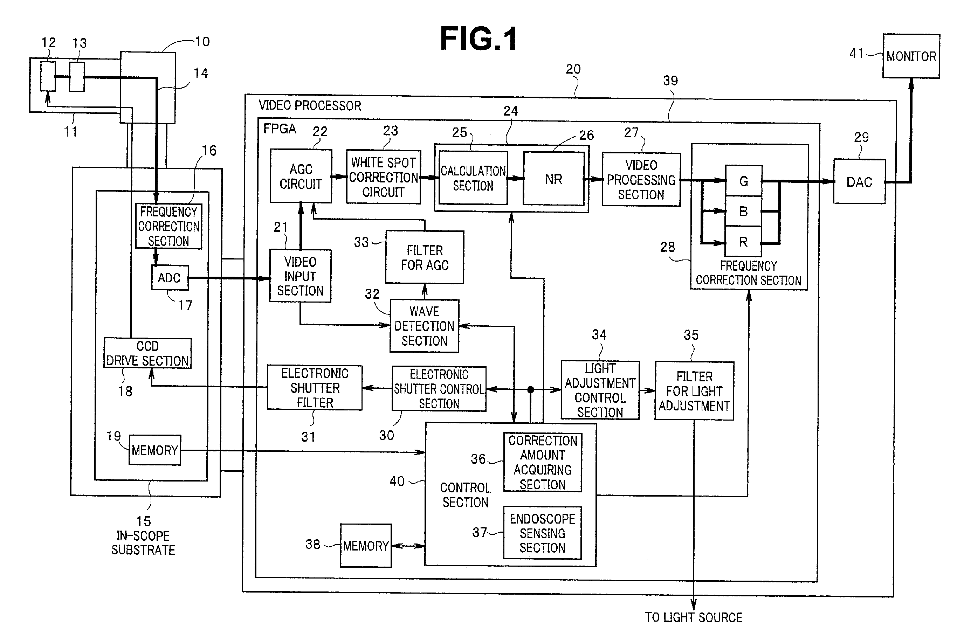

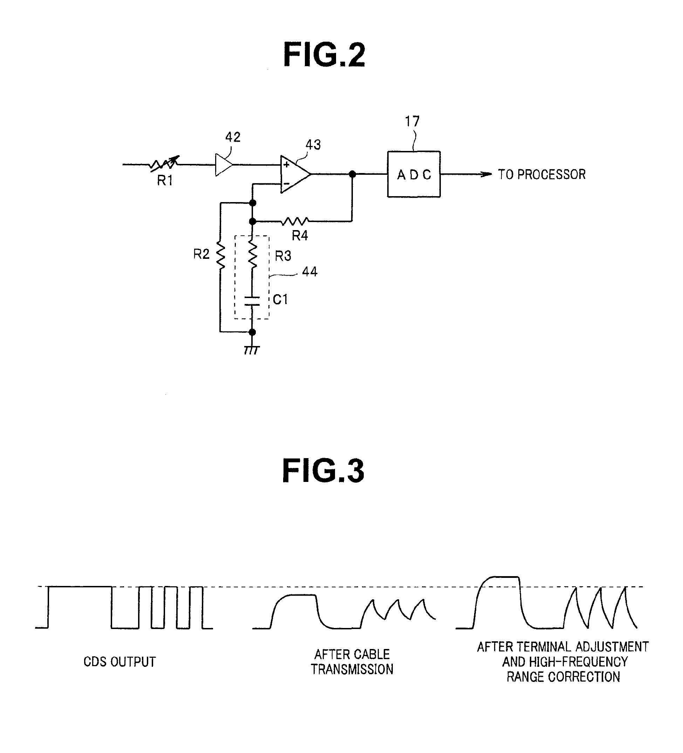

Fig. 1 is a block diagram showing an endoscope system according to a first embodiment of the present invention. Fig. 2 is a circuit diagram showing a specific configuration of a frequency correction section 16 of an in-scope substrate 15 in Fig. 1. Fig. 3 is a waveform chart for explaining action of the frequency correction section 16. Note that bold lines in Fig. 1 indicate a flow of a video signal.

The endoscope system shown in Fig. 1 is composed of an endoscope 10 and a video processor 20. In the endoscope 10, an image pickup section 12 is provided at a distal end of an insertion section 11. The image pickup section 12 photoelectrically converts an optical image from an object and outputs a video signal of the object to a CDS circuit section 13. The CDS circuit section 13 subjects the video signal outputted from the image pickup section 12 to CDS (correlated double sampling) processing.

The video signal from the CDS circuit section 13 is transferred to the frequency correction section 16 of the in-scope substrate 15 via a cable 14 which is routed through the endoscope 10. The frequency correction section 16 as an in-scope correction circuit performs frequency correction processing on the inputted video signal and then outputs the video signal to an AD converter (ADC) 17. The ADC 17 converts the inputted analog video signal to a digital signal and outputs the digital signal.

In Fig. 2, the frequency correction section 16 is composed of an operational amplifier 43. The video signal from the CDS circuit section 13 is supplied to a positive-phase input terminal of the operational amplifier 43 via a terminating resistor R1 and a buffer 42. An output terminal of the operational amplifier 43 is connected to a negative-phase input terminal via a resistor R4. The negative-phase input terminal of the operational amplifier 43 is connected to a reference potential point via a high-frequency range correction control section 44 which is composed of a capacitor C1 and a resistor R3, and a resistor R2 is connected in parallel to the high-frequency range correction control section 44.

The frequency correction section 16 with the above-described configuration can perform gain control and high-frequency range enhancement by adjustment of a resistance of the terminating resistor R1 and the high-frequency range correction control section 44. A left-side portion of Fig. 3 shows a waveform of CDS output from the CDS circuit section 13. The CDS output has a pulsed waveform corresponding to respective pixels, and pulse amplitudes correspond to respective levels of the pixels.

The example in Fig. 3 shows an example of CDS output with same pixel levels. The CDS output attenuates according to cable transmission loss at the time of transmission through the cable 14. A central portion in Fig. 3 shows a waveform after the transmission through the cable 14. The example in Fig. 3 shows that a high-frequency range component, in particular, of the CDS output has been attenuated by the cable 14. The CDS output transmitted through the cable 14 is caused by adjustment of the terminating resistor R1 and high-frequency range correction of the frequency correction section 16 to have a waveform shown on a right side of Fig. 3. As shown in Fig. 3, the CDS output has higher levels and a slightly improved high-frequency range characteristic.

Note that the frequency correction section 16 cannot always perform sufficient frequency correction, as shown in Fig. 3. The frequency correction section 16 performs frequency correction for suppressing deterioration in resolution such that sufficient frequency correction processing can be performed in the video processor 20. After such an output from the frequency correction section 16 is converted to a digital signal by the ADC 17, the digital signal is supplied to the video processor 20.

A CCD drive section 18 is also provided in the in-scope substrate 15. The CCD drive section 18 is configured to drive a CCD constituting the image pickup section 12 in accordance with a control signal from the video processor 20. Note that the image pickup section 12 may be composed of an image pickup device other than a CCD.

In the present embodiment, a memory 19 is provided in the in-scope substrate 15. The memory 19 stores scope information related to the endoscope 10. Examples of the scope information include information on the type of the endoscope 10 and cable length and cable transmission characteristics of the cable 14 and information on the image pickup section 12. For example, the scope information includes information on variation in sensitivity of the image pickup device such as the CCD constituting the image pickup section 12 and information such as a correction value for correcting variation in sensitivity among pixels. The scope information further includes information on a gain value if an image pickup device whose gain can be adjusted is used as the image pickup section 12. The video processor 20 is configured to be capable of reading out and acquiring the scope information stored in the memory 19.

Note that although an example in which the scope information is stored in the memory 19 is described in the present embodiment, one or more resistors having a resistance corresponding to the scope information may be provided in the endoscope 10, and the scope information may be acquired when the video processor 20 reads out the resistance.

The video processor 20 is composed of an FPGA (field-programmable gate array) 39 and a DAC 29. A video input section 21 in the video processor 20 takes in the video signal from the endoscope 10 and outputs the video signal to an AGC circuit 22 and a wave detection section 32. The AGC circuit 22, whose gain is controlled by an output from a filter 33 for AGC (to be described later), amplifies the inputted video signal and outputs the video signal to a white spot correction circuit 23 which is one of in-processor correction circuits.

In fluorescence imaging, since a high-sensitivity image pickup device is used, a pixel defect called a white spot may appear in an observed image. Methods for correcting a white spot include a process of determining whether a pixel of interest is a white spot by comparing the pixel of interest with an average value of luminance signals of pixels surrounding the pixel of interest and, if it is determined that the pixel of interest is a white spot, replacing the pixel of interest with the average value of the surrounding pixels. However, the process suffers from the problem of deterioration in resolution. Accordingly, the white spot correction circuit 23 detects a white spot pixel by using a second largest one of pixel values of pixels surrounding a pixel of interest.

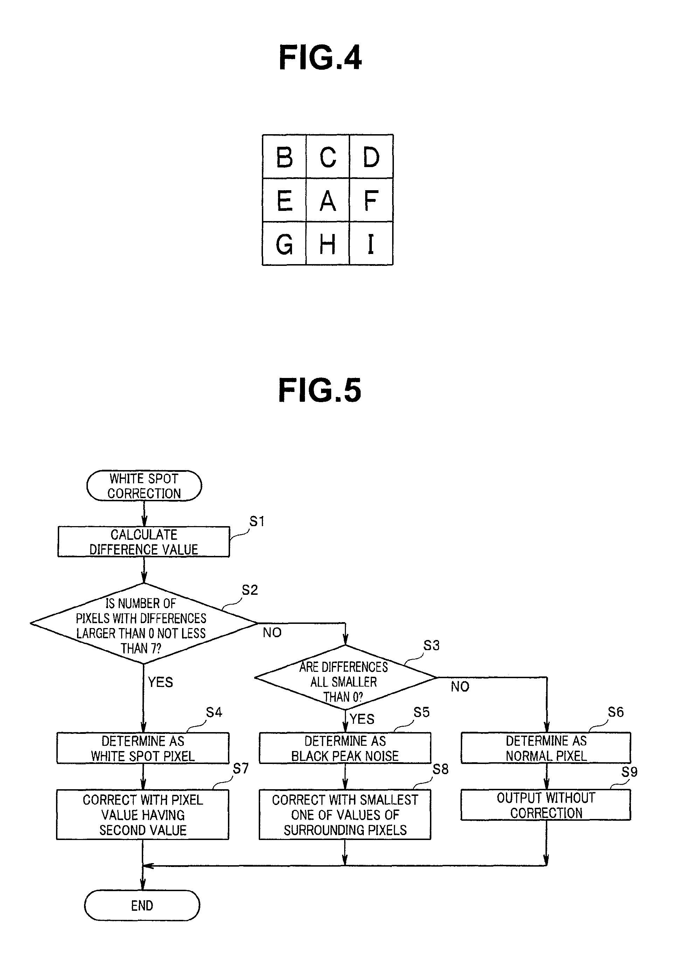

Fig. 4 is an explanatory view for explaining operation of the white spot correction circuit 23. Fig. 5 is a flow chart showing an operation flow of the white spot correction circuit 23.

The white spot correction circuit 23 performs correction of a white spot using a pixel A of interest at a center in Fig. 4 and pixels B to I surrounding the pixel A of interest. The white spot correction circuit 23 detects a pixel with a second largest one (hereinafter referred to as a second value) of respective pixel values of the surrounding pixels B to I of 3 × 3 pixels. For example, assume a case with same values. If the eight pixels values of the surrounding pixels B to I are (7, 7, 6, 5, 4, 3, 2, and 1) (hereinafter referred to as Example 1) in descending order, a second value is not '6' but '7.'

In step S1 of Fig. 5, the white spot correction circuit 23 obtains a difference value between the pixel A of interest and each of the surrounding pixels B to I by the calculation "pixel of interest - surrounding pixel. " In step S2, then, the white spot correction circuit 23 determines whether the number of difference values larger than 0 of obtained eight difference values is not less than 7. In Example 1 above, if a pixel value of the pixel A of interest is not less than 8, the white spot correction circuit 23 shifts to step S4 to determine that the pixel A of interest is a white spot pixel.

The condition that the number is not less than 7 allows an image pickup device with two white spots in a region of 3 × 3 pixels to be set as an object to be corrected.

If the number of difference values larger than 0 is less than 7, the white spot correction circuit 23 determines in a next step, S3 whether the difference values are all smaller than 0. If the pixel value of the pixel A of interest is 0 in Example 1 above, the white spot correction circuit 23 shifts to step S5 to determine that the pixel A of interest is black peak noise. If the pixel value of the pixel A of interest is not less than 1 in Example 1 above, the white spot correction circuit 23 shifts to step S6 to determine that the pixel A of interest is a normal pixel.

If the white spot correction circuit 23 determines that the pixel A of interest is a white spot pixel, the white spot correction circuit 23 shifts to step S7 to correct the pixel A of interest with a second value. If the white spot correction circuit 23 determines that the pixel A of interest is black peak noise, the white spot correction circuit 23 shifts to step S8 to correct the pixel A of interest with a smallest value of the surrounding pixels. If the white spot correction circuit 23 determines that the pixel A of interest is a normal pixel, the white spot correction circuit 23 shifts to step S9 to output the pixel A of interest without correction.

As described above, if the white spot correction circuit 23 is used, a white spot can be corrected by reliably detecting a white spot pixel and replacing the white spot pixel with a second value.

Note that the white spot correction circuit 23 may be configured so as to take a black peak pixel into consideration. If eight results obtained by the difference value calculation "pixel of interest - surrounding pixel" are all smaller than 0, a pixel of interest can be detected as a black peak pixel. In the case, the black peak pixel can be corrected by replacing the pixel of interest with a second value.

The video signal, a white spot of which has been corrected by the white spot correction circuit 23, is given to a gain correction circuit 24 which is one of the in-processor correction circuits. In the gain correction circuit 24, a calculation section 25 and a noise reduction circuit (NR) 26 are provided. The gain correction circuit 24 corrects gain of a video signal under control of a control section 40.

The control section 40 reads out the scope information from the memory 19 in the endoscope 10. The scope information includes a gain correction value corresponding to variation of a CCD as the image pickup section 12. A correction amount acquiring section 36 of the control section 40 acquires the gain correction value. The control section 40 controls the gain correction circuit 24 on the basis of the gain correction value included in the scope information.

The calculation section 25 of the gain correction circuit 24 corrects variation in sensitivity of the CCD by, e.g., multiplying a video signal by the gain correction value. The calculation processing by the calculation section 25 allows correction of variation in sensitivity of the image pickup device as the image pickup section 12 provided in the endoscope 10.

An output from the calculation section 25 is given to the noise reduction circuit (NR) 26. The NR 26 removes noise in the video signal from the calculation section 25 and then outputs the video signal to a video processing section 27. Although the calculation by the calculation section 25 may increase a noise component, since the NR 26 is arranged downstream of the calculation section, an increase in noise component can be reliably removed.

The NR 26 may also change noise reduction strength according to the gain correction value by which a video signal is to be multiplied by the calculation section 25. For example, the NR 26 increases the noise reduction strength if the gain correction value is larger than 1 and reduces the noise reduction strength if the gain correction value is smaller than 1.

In the present embodiment, a light adjustment control section 34 is also provided as an in-processor correction circuit. The light adjustment control section 34 outputs a light adjustment control signal to a light source (not shown) via a filter 35 for light adjustment under control of the control section 40. For example, the insertion section 11 of the endoscope 10 includes, at the distal end, an optical system (not shown) which illuminates an object with light from the light source. Level of a video signal from the image pickup section 12 can be changed by changing the amount of illuminating light from the light source. For the reason, variation in sensitivity of the CCD can be corrected by controlling the amount of illuminating light from the light source according to the variation in sensitivity.

The light adjustment control section 34 is given a sensitivity correction value included in the scope information by the control section 40 and outputs the light adjustment control signal for correcting variation in sensitivity of the CCD and a synchronization signal corresponding to the CCD to the light source. The light adjustment control signal is supplied to the light source via the filter 35 for light adjustment, which controls the amount of illuminating light according to sensitivity of the CCD. This allows correction of variation in sensitivity of the CCD. The light adjustment control section 34 stops outputting a synchronization signal until the light adjustment control section 34 senses a connected CCD at the time of activation of the video processor and outputs a synchronization signal corresponding to the sensed CCD after the sensing. After that, if a scope is removed, and no CCD is connected, the synchronization signal corresponding to the CCD that had been outputted before the removal of the scope is outputted. If the light adjustment control section 34 senses a new CCD after the removal of the scope, the light adjustment control section 34 outputs a synchronization signal corresponding to the new CCD.

The gain correction circuit 24 and the light adjustment control section 34 may also be configured to correct variation in sensitivity on the basis of not only the scope information stored in the endoscope 10 but also a video signal inputted to the video processor 20.

The wave detection section 32 detects a wave in the video signal from the video input section 21 and outputs a wave detection result of the video signal to the control section 40. Video signals during a period (OB period) corresponding to optical black are noise components. The control section 40 adds up noise signals during the OB period and calculates an average value (hereinafter referred to as an average OB value) of signal levels during the OB period. If the sensitivity of the CCD is high, the average OB value is relatively high. On the other hand, if the sensitivity of the CCD is low, the average OB value is relatively low. Accordingly, variation in sensitivity of the CCD can be detected from the average OB value. That is, the control section 40 gives a correction value based on the average OB value to the gain correction circuit 24 and the light adjustment control section 34, which allows the gain correction circuit 24 and the light adjustment control section 34 to correct variation in sensitivity of the CCD. For example, the control section 40 can use, as a correction value, a value obtained by dividing a predetermined value by the average OB value.

Also, the control section 40 controls the amount of illuminating light from the light source in order to determine a target value for correcting variation in sensitivity. For example, the control section 40 calculates average luminance on the basis of the output from the wave detection section 32. The control section 40 controls the light adjustment control section 34 to adjust the amount of illumination such that the target correction value obtained by multiplying a predetermined target value by a correction value is close to the average luminance.

Additionally, the control section 40 may stop calculation operation of the calculation section 25 until a difference between the target correction value and the average luminance exceeds a predetermined threshold value. That is, the control section 40 may cause the calculation section 25 to start calculation operation and suppress variation in sensitivity if the variation in sensitivity becomes relatively large, and the target correction value deviates from the average luminance by a value larger than the predetermined threshold value.

A noise-reduction effect of the NR 26 changes depending on the type of the CCD constituting the image pickup section 12, observation mode, gain, and the like. Accordingly, the control section 40 is configured to control noise reduction by the NR 26 according to the factors.

For example, if the image pickup section 12 is composed of an image pickup device whose gain value can be changed, the correction amount acquiring section 36 of the control section 40 extracts information on the gain value of the image pickup device from the scope information, and the control section 40 controls noise reduction by the NR 26 according to the gain value. The control section 40 is configured to increase the noise reduction strength with an increase in gain value.

Note that if an image pickup section of a scope connected to the video processor 20 is driven under control of the control section 40, as in the endoscope 10 in Fig. 1, a gain value of an image pickup device is known to the control section 40. Accordingly, in the case, the control section 40 may acquire the gain value without scope information and control the NR 26.

For example, the NR 26 holds a plurality of types of noise reduction parameters. The control section 40 can determine the noise reduction strength by specifying one to be used of the noise reduction parameters. For example, the control section 40 can set the noise reduction strength so as to correspond to a gain value by classifying gain values into n (e.g., 8) levels and specifying one of noise reduction parameters for the n (e.g., 8) levels.

For example, if the NR 26 is configured to have a smoothing filter, the control section 40 can obtain the noise reduction strength corresponding to the gain value by setting a strength parameter for the smoothing filter according to the gain value.

Moreover, the control section 40 may change the noise reduction strength according to the amount of noise included in a video signal. For example, the control section 40 obtains the amount of noise during the OB period from the output from the wave detection section 32 and changes the noise reduction strength according to the obtained noise amount. In the case as well, the control section 40 may classify noise amounts into n levels and control the noise reduction strength in n levels.

Furthermore, the control section 40 may change the noise reduction strength according to the type of the CCD. For example, the correction amount acquiring section 36 of the control section 40 acquires resolution of the CCD from the scope information and increases the noise reduction strength with an increase in resolution.

The control section 40 may also change the noise reduction strength according to the type of the light source. For example, illuminating light from the light source changes according to mode, such as normal-light observation mode, fluorescence observation mode, narrow band observation mode, or infrared observation mode. Accordingly, the control section 40 changes the noise reduction strength according to observation mode.

The video processor 20 is also capable of displaying external input inputted via an external input terminal (not shown) as a picture-in-picture. In the case, the control section 40 may change the noise reduction strength for an externally inputted video signal.

The video processor 20 is also configured to control brightness of an inputted video signal. As examples of brightness control, gain control by the AGC circuit 22, light adjustment control by the light adjustment control section 34, and electronic shutter control by an electronic shutter control section 30 are conceivable.

A wave detection result from the wave detection section 32 is supplied as an AGC control signal to the AGC circuit 22 via the filter 33 for AGC. The AGC circuit 22 controls gain of an inputted video signal in accordance with the AGC control signal such that average luminance level is a predetermined level. The control section 40 is configured to control the wave detection section 32 to set a target brightness value.

As described above, the light adjustment control section 34 performs light adjustment control on the light source under control of the control section 40. The control section 40 can set the target brightness value for light adjustment control by the light adjustment control section 34.

The electronic shutter control section 30 generates an electronic shutter control signal for controlling opening and closing of an electronic shutter under control of the control section 40. The electronic shutter control signal is given to the CCD drive section 18 via an electronic shutter filter 31. Opening and closing of the electronic shutter of the image pickup section 12 is controlled by the electronic shutter control signal. The control section 40 can set the target brightness value for electronic shutter control in the electronic shutter control section 30.

In the present embodiment, the AGC control signal from the wave detection section 32, the light adjustment control signal from the light adjustment control section 34, and the electronic shutter control signal from the electronic shutter control section 30 are supplied to the AGC circuit 22, the light source, and the CCD drive section 18, respectively, via the filter 33 for AGC, the filter 35 for light adjustment, and the electronic shutter filter 31.

Accordingly, time constants for transient response can be adjusted by appropriately setting filter factors of the filters 33, 35, and 31. That is, response speeds of AGC control, light adjustment control, and electronic shutter control can be controlled by the filters 33, 35, and 31. Note that the filter factors of the filters 33, 35, and 31 may be configured to be changeable by the control section 40.

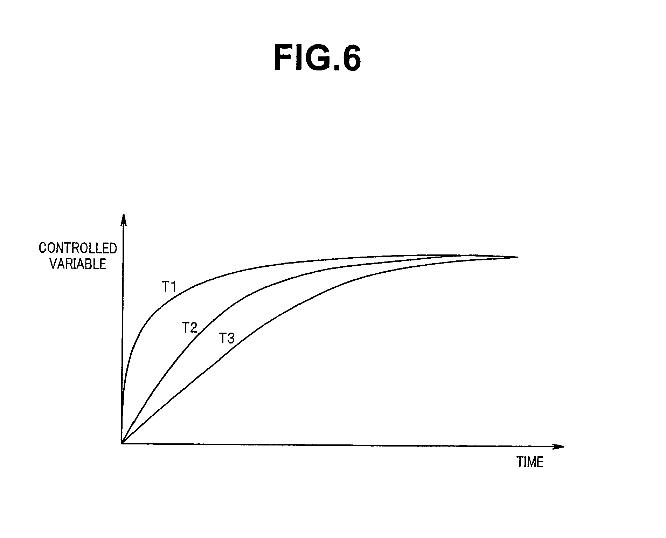

Fig. 6 is a graph showing transient response characteristics, with time on an abscissa and a controlled variable on an ordinate. Time constants T1 to T3 in Fig. 6 have the relation T1 < T2 < T3. For example, let T1 be a time constant of the electronic shutter filter 31; T2, a time constant of the filter 35 for light adjustment; and T3, a time constant of the filter 33 for AGC.

In the case, under control of the control section 40, the electronic shutter is first controlled to change brightness, the light source is then controlled to change brightness, and the AGC circuit 22 is finally controlled to change brightness. In the case, for example, the control section 40 sets a target value for brightness control by the electronic shutter to a highest value, sets a target value for brightness control by the light source to a second highest value, and sets a target value for brightness control by the AGC circuit 22 to a lowest value.

In the description of Fig. 6, an example in which the response speed of electronic shutter control is set so as to be highest, the response speed of light adjustment control is set so as to be lower, and the response speed of AGC control is set so as to be still lower has been described. The response speed of light adjustment control may be set so as to be highest, the response speed of electronic shutter control may be set so as to be lower, and the response speed of AGC control may be set so as to be still lower. In the case, the target value for brightness control by light adjustment may be set so as to be highest, the target value for brightness control by electronic shutter control may be set so as to be lower, and the target value for brightness control by AGC control may be set so as to be still lower.

The video signal from the gain correction circuit 24 is supplied to a video processing section 27. The video processing section 27 subjects the inputted video signal to y correction processing and white balance adjustment processing and then outputs the video signal to a frequency correction section 28. The frequency correction section 28 performs frequency correction on the video signal from the video processing section 27 for each combination of R, G, and B signals and frequency bands.

A memory 38 stores various types of correction information (correction values) used in the in-processor correction circuits for each scope type. In the video processor 20, the memory 38 stores correction information for correction that takes into consideration correction in the in-scope correction circuit.

For example, the memory 38 stores, for each scope, frequency correction information for each combination of frequency bands and R, G, and B signals. Frequency correction information to be stored by the memory 38 gives a frequency correction amount for performing frequency correction in concert with frequency correction by the frequency correction section 16 that is an in-scope correction circuit.

As described above, the control section 40 reads out the scope information from the memory 19 of the endoscope 10. An endoscope sensing section 37 of the control section 40 senses the type of an endoscope connected to the video processor 20 from scope information. The endoscope sensing section 37 can recognize the type of a scope connected to the video processor 20 e.g., whether the scope is an analog scope or a digital scope, whether the scope incorporates a CDS circuit section, or what correction circuit the scope incorporates as an in-scope correction circuit.

The correction amount acquiring section 36 acquires correction information for correction in the in-processor correction circuits on the basis of the scope information. For example, the correction amount acquiring section 36 reads out frequency correction information from the memory 38. With the information, the control section 40 can set, in the frequency correction section 28, a frequency characteristic to be corrected on the video processor 20 side.

The frequency correction section 28 performs frequency correction on the basis of correction information from the control section 40. That is, the frequency correction section 28 performs frequency correction according to endoscope type for each combination of R, G, and B signals and frequency bands, in concert with frequency correction by the frequency correction section 16 that is an in-scope correction circuit.

As described above, the frequency correction section 28 performs frequency correction on the basis of correction information from the control section 40. This allows frequency correction in concert with frequency correction by the frequency correction section 16 that is an in-scope correction circuit and allows extremely effective frequency correction. Frequency correction processing by the frequency correction section 28 performs edge enhancement and suppresses deterioration in resolution.

Note that the degree of deterioration in an image at the time of transmission through the cable 14 is different between a horizontal direction and a vertical direction of the image. For the reason, the correction amount acquiring section 36 may acquire information on horizontal and vertical resolutions and pieces of correction information for horizontal and vertical directions on the basis of the scope information, the control section 40 may give the respective pieces of correction information for the horizontal direction and the vertical direction to the frequency correction section 28, and the frequency correction section 28 may perform frequency correction independently for the horizontal direction and the vertical direction.

An output from the frequency correction section 28 is given to the DA converter (DAC) 29. The DAC 29 restores an inputted video signal to an analog signal and outputs the analog signal to a monitor 41. In the above-described manner, video based on the picked-up image from the endoscope 10 is displayed on a display screen of the monitor 41.

Note that the frequency correction section 16 may also perform frequency correction for each combination of frequency bands, R, G, and B signals, and horizontal and vertical directions, like the frequency correction section 28 in the video processor 20.

Operation of the embodiment with the above-described configuration will be described.

The endoscope 10 is connected to the video processor 20, and the endoscope 10 and the video processor 20 are turned on. This causes the CCD drive section 18 of the endoscope 10 to drive the CCD as the image pickup section 12 to pick up an image of an object. A video signal from the image pickup section 12 is subjected to CDS processing in the CDS circuit section 13 and is supplied to the frequency correction section 16 on the in-scope substrate 15 via the cable 14. The frequency correction section 16 performs frequency correction for high-frequency range enhancement to compensate for attenuation by the cable 14. Note that although the frequency correction section 16 cannot perform sufficient frequency correction, the frequency correction section 16 prevents deterioration in resolution and allows efficient frequency correction processing in the video processor 20. The video signal from the frequency correction section 16 is converted to a digital signal in the ADC 17 and is then outputted to the video processor 20.

The control section 40 of the video processor 20 reads out the scope information stored in the memory 19 of the endoscope 10. The correction amount acquiring section 36 of the control section 40 acquires various types of correction information for respective correction circuits in the video processor 20 on the basis of the scope information. For example, the correction amount acquiring section 36 reads the memory 38 on the basis of the scope information and acquires pieces of frequency correction information for respective frequency bands corresponding to the type of the endoscope 10 connected to the video processor 20. The control section 40 controls the respective sections on the basis of the respective pieces of correction information acquired by the correction amount acquiring section 36.

The video signal from the endoscope 10 is supplied to the AGC circuit 22 via the video input section 21. The wave detection section 32 detects a wave in the inputted video signal and supplies an AGC control signal to the AGC circuit 22 via the filter 33 for AGC. The AGC circuit 22 amplifies the video signal with a gain based on the AGC control signal and then outputs the video signal to the gain correction circuit 24. Since a gain correction value has been given from the control section 40 to the gain correction circuit 24, the calculation section 25 corrects variation in sensitivity by, e.g., multiplying the video signal by the gain correction value. The video signal from the calculation section 25 is cleared of noise by the NR 26 and is then supplied to the video processing section 27.

The video processing section 27 subjects the inputted video signal to predetermined video signal processing and then outputs the video signal to the frequency correction section 28. In the present embodiment, the pieces of frequency correction information based on the scope information are supplied from the control section 40 to the frequency correction section 28. That is, the pieces of frequency correction information supplied to the frequency correction section 28 are intended for performing processing in concert with frequency correction processing in the frequency correction section 16 of the in-scope substrate 15, and the frequency correction section 28 can perform optimum frequency correction processing in combination with the frequency correction processing in the frequency correction section 16.

The video signal which has undergone optimum frequency correction by the frequency correction section 28, edge enhancement, and suppression of deterioration in resolution is supplied to the DAC 29. The DAC 29 restores the inputted video signal to an analog signal and then outputs the analog signal to the monitor 41. In the above-described manner, the picked-up image based on the video signal optimized by various types of correction in the video processor 20 that take into consideration various types of correction processing in the endoscope 10 is displayed on the display screen of the monitor 41.

As described above, in the present embodiment, transmission of correction information in an in-scope correction circuit to a video processor allows the video processor to perform correction processing in concert with correction processing by the in-scope correction circuit using correction information that takes into consideration the correction information in the in-scope correction circuit. This allows optimum correction processing on a video signal and obtainment of a high-quality picked-up image.

Note that although an example in which only a frequency correction section is adopted as an in-scope correction circuit has been described in the present embodiment, various types of correction circuits, such as a gain correction circuit and a circuit for correcting variation in sensitivity, may be apparently adopted as in-scope correction circuits. Even in the case, a correction amount acquiring section reads out correction information for performing correction in concert with correction processing by the in-scope correction circuits from a memory in a video processor.

Correction information which is used in an in-video-processor correction circuit and takes into consideration correction by an in-scope correction circuit has been described as being stored in a memory in a video processor in the present embodiment. However, correction information to be used alone in an in-video-processor correction circuit may be stored in the memory in the video processor, and the correction information read out from the memory on the basis of scope information by a correction amount acquiring section may be corrected to correction information which takes into consideration correction by an in-scope correction circuit.

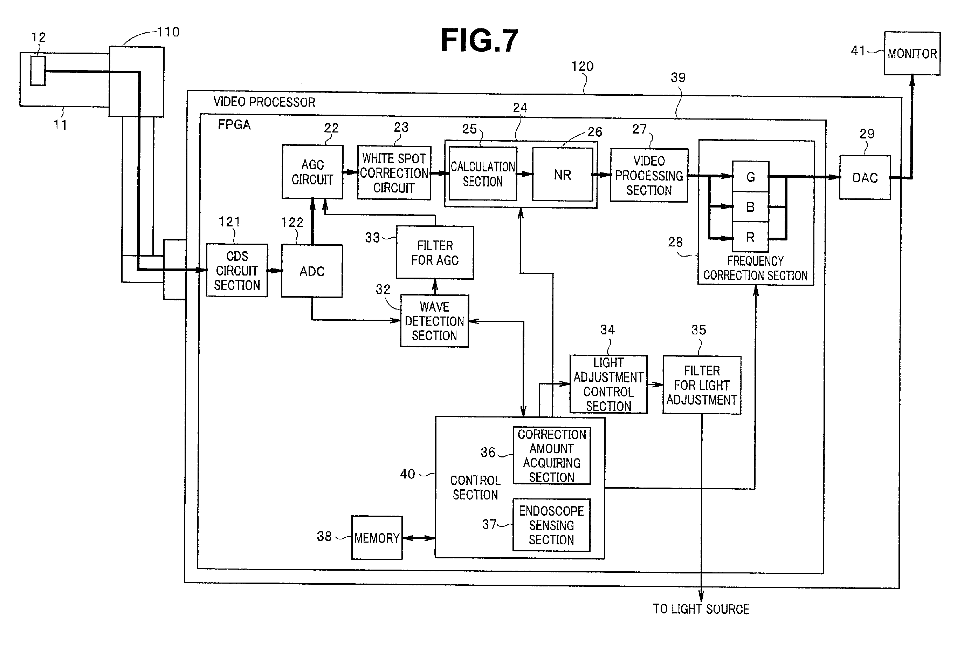

Fig. 7 is a block diagram showing a second embodiment of the present invention. Same components in Fig. 7 as the components in Fig. 1 are denoted by same reference numerals, and a description of the components will be omitted.

The present embodiment is different from the first embodiment in that a video processor 120 to which an analog endoscope 110 is connected is adopted. The analog endoscope 110 outputs a video signal from an image pickup section 12 to the video processor 120.

The video processor 120 is different from the video processor 20 according to the first embodiment only in that a CDS circuit section 121 and an ADC 122 are adopted instead of the video input section 21 and that the electronic shutter control section 30 and the electronic shutter filter 31 1 are omitted.

The CDS circuit section 121 subjects the video signal from the endoscope 110 to CDS processing and then outputs the video signal to the ADC 122. The ADC 122 converts the inputted video signal to a digital signal and outputs the digital signal to an AGC circuit 22 and a wave detection section 32.

In the embodiment with the above-described configuration, when the endoscope 110 is connected to the video processor 120, and the endoscope 110 and the video processor 120 are turned on, a video signal from the endoscope 110 is supplied to the video processor 120. A control section 40 of the video processor 120 senses, by an endoscope sensing section 37, that the endoscope 110 that outputs an analog signal is connected, that a video signal inputted to the video processor 120 has not been corrected by an in-scope correction circuit, and that scope information is not provided.

Correction information necessary for performing correction processing on a video signal by the video processor 120 alone is stored in a memory 38. In the case, the control section 40 reads out the correction information for performing correction processing by the video processor 120 alone which is stored in the memory 38 and controls an in-video-processor correction circuit.

Note that if information for determining the type of an endoscope is provided from an endoscope, the control section 40 reads out correction information for each endoscope type which is stored in the memory 38 and is intended for correction processing by the video processor 120 alone and supplies the correction information to respective correction circuits.

As described above, the present embodiment can subject a video signal to optimum correction processing by reading out correction information from memory even if an analog endoscope is connected to a video processor. Other effects are same as the effects of the first embodiment.

The present application is filed claiming the priority of Japanese Patent Application No. 2010-173862 filed in Japan on August 2, 2010, and the above described disclosure is incorporated by reference in the present description, claims and drawings.

## Claims

1. An endoscope system comprising: an endoscope which outputs a video signal based on an endoscope image obtained by an image pickup section for picking up an optical image of a subject; and a video processor to which the endoscope is detachably connected and which performs video signal processing on the video signal from the endoscope,
wherein the video processor comprises
a correction amount acquiring section, in a case where the endoscope has a first frequency correction section for performing a frequency correction of the video signal, to which first frequency correction information used in the first frequency correction section is inputted and which acquires second frequency correction information for performing a frequency correction in concert with frequency correction processing by the first frequency correction section based on the first frequency correction information, and
a second frequency correction section which performs the frequency correction on the video signal from the endoscopes using the second frequency correction information.

2. The endoscope system according to claim 1, wherein
the video processor comprises an endoscope sensing section which senses whether or not the endoscope has the first frequency correction section for performing the frequency correction of the video signal,
in the case where the endoscope has the first frequency correction section, the correction amount acquiring section gives the second frequency correction information to the second frequency correction section and causes the second frequency correction section to perform the frequency correction, and
in a case where the endoscope does not have the first frequency correction section, the correction amount acquiring section acquires third frequency correction information for the second frequency correction section to perform the frequency correction alone and gives the third frequency correction information to the second frequency correction section and causes the second frequency correction section to perform the frequency correction.

3. The endoscope system according to claim 1, wherein
the video processor has a memory which stores the second frequency correction information.

4. The endoscope system according to claim 2, wherein
the video processor has a memory which stores the second and third frequency correction information.

5. The endoscope system according to claim 1 or 2, wherein
the video processor has a memory which stores the third frequency correction information for the second frequency correction section to perform the frequency correction alone, and
the correction amount acquiring section generates the second frequency correction information based on the first frequency correction information and the third frequency correction information.

6. The endoscope system according to claim 1 or 2, wherein
the first frequency correction section corrects transmission loss of the video signal caused by a transmission channel extending from the image pickup section to the first frequency correction section, and
the second frequency correction section performs edge enhancement processing on the video signal.

7. The endoscope system according to claim 1 or 2, wherein
the second frequency correction section performs frequency correction on the video signal for each combination of types of endoscopes connected to the video processor and frequency bands.

8. The endoscope system according to claim 1 or 2, wherein
the second frequency correction section performs frequency correction independently for a horizontal direction and a vertical direction of the endoscope image.

9. The endoscope system according to claim 1, wherein
the endoscope has a first information memory which stores the first frequency correction information.

10. The endoscope system according to claim 9, wherein
the video processor reads the first frequency correction information from the first information memory when the endoscope is connected.

11. The endoscope system according to claim 1, wherein
the video processor comprises a gain correction section to which scope information corresponding to sensitivity of the image pickup section is inputted and which amplifies the video signal from the endoscope with a gain correction value based on the inputted scope information and thereafter gives the amplified video signal to the second frequency correction section.

12. The endoscope system according to claim 10, wherein
the gain correction section comprises a noise reduction section which performs noise reduction processing on an output of the gain correction section with noise reduction strength based on the gain correction value.

13. The endoscope system according to claim 1, wherein
the video processor comprises a light adjustment control section to which scope information corresponding to sensitivity of the image pickup section is inputted and which adjusts an amount of illuminating light for illuminating the subject based on the inputted scope information.
